# EUROPEAN PATENT APPLICATION

(11) **EP 2 060 281 A1**
(43) Date of publication of application: **20.05.2009**
(21) Application number: 07120526.4
(22) Date of filing: 13.11.2007
(51) Int. Cl.: A61M 1/00

(54) **A vacuum regulator valve**

(71) Applicant: Iberhospitex S.A., 08185 Lliça de Vall (Barcelona) (ES)
(72) Inventor: Doucastella Codina, Lluís, 08185 Lliçà de Vall (Barcelona) (ES); Idelsohn Zielonka, Sebastián, 08185 Lliçà de Vall (Barcelona) (ES); Fernandez Sanchez, David, 08185 Lliçà de Vall (Barcelona) (ES); Lopez Rodriguez, Aniceto, 08185 Lliçà de Vall (Barcelona) (ES)
(74) Representative: Barlocci, Anna

(57) **Abstract**

It comprises a variable volume chamber (2) having a first opening (3) connectable to a vacuum source (40) and a second opening (4) connectable to a suction conduit (52; 36), such that, in use, the chamber (2) may be put in fluid communication with the vacuum source (40) and vacuum may be generated in the chamber to cause suction through the suction conduit (52; 36); the valve comprises a bellows (1; 1a) with at least one pleat, arranged to define the chamber (2) and operable to move, in use, between an expanded condition in which the first opening (3) of the chamber (2) remains open, and a contracted condition in which it causes the closure of the first opening (3). It constitutes a very simple and trouble-free device for draining a wound at a relatively low vacuum level using a high vacuum suction bottle.

## Description

The present invention relates to a vacuum regulator valve, which can be employed, for example, to drain a wound by means of a high vacuum suction bottle, when it is desirable to perform the drainage operation at a relatively low vacuum level.

### BACKGROUND OF THE INVENTION

Known in the art are pre-evacuated high vacuum suction bottles for removing body fluids, for example for draining a wound after surgery. Such bottles may have a vacuum level e.g. of 5% of the atmospheric pressure, predetermined by the manufacturer, and are connected to the wound by means of a suction tube with a perforated end portion which is arranged in the cavity of the wound; they withdraw body fluids at a relatively constant vacuum level.

However, the high vacuum of such bottles is not suitable for all medical situations; in some cases, such as fresh wounds, the suction force provided by the high vacuum in the bottle is too strong, and it is desirable to use a regulator device.

Some known regulator devices or valves for reducing the suction force are connected to the suction bottle at one end, and to the fluid source at the other, and adjust the vacuum level in the suction tube by mechanically obstructing the passage between the bottle and the wound, or in general between the vacuum source and the source of body fluid. They provide a relatively constant vacuum level in the suction tube which is lower than the level inside the suction bottle.

One such valve is disclosed for example in US5073172. The valve device comprises an elastic diaphragm attached to the inner wall of a cylindrical housing, defining a variable volume chamber with a first opening connected to the bottle and a second opening connected to the suction conduit. The bottle and the suction conduit are in communication through the chamber when the two openings remain open. However, due to the high vacuum in the bottle, the elastic diaphragm can close the first opening, isolating the chamber from the bottle: in this condition, the level of vacuum in the chamber decreases and a lower suction force is exerted on the wound through the suction conduit. In practice, the elastic diaphragm operates to successively open and close the first opening.

However, this known device is relatively complex to manufacture and to assemble: for example, it must have a holder and sealing means between the diaphragm and the housing. It is desirable to provide a simple and versatile device that can be satisfactorily employed to reduce the suction force exerted by the high vacuum source on the wound.

### DESCRIPTION OF THE INVENTION

According to one aspect, the present invention provides a vacuum regulator valve comprising a variable volume chamber having a first opening connectable to a vacuum source and a second opening connectable to a suction conduit, such that, in use, the chamber may be put in fluid communication with the vacuum source and vacuum may be generated in the chamber to cause suction through the suction conduit, characterised in that the valve comprises a bellows with at least one pleat, arranged to define the chamber and operable to move, in use, between an expanded condition in which the first opening of the chamber remains open, and a contracted condition in which it causes the closure of the first opening.

The use of a bellows to define the variable volume chamber allows obtaining a very simple and trouble-free device, which can be manufactured easily and at low cost; for example, in some embodiments it can be manufactured by a single moulding operation in which the chamber and openings can be formed in one piece using an appropriate elastomeric material.

When the device is connected to a high vacuum bottle, the vacuum level is maintained substantially constant throughout the drainage operation, regardless of the filling level of the bottle.

Preferably, in the contracted condition of the bellows a portion of its inner wall rests against the first opening closing it; this has the advantage that no additional closing element is needed.

In one embodiment, the portion of the inner wall of the bellows which closes the first opening may comprise a thickened section, in order to guarantee an optimum closure between the wall of the bellows and the first opening.

According to an embodiment, the bellows comprises two substantially flat end walls and a side wall which is at least partly pleated.

The first opening and second opening may be defined on the same flat end wall, but they can also be defined on opposite flat end walls; in other embodiments, the first opening may be defined on one flat end wall and the second opening may be defined on the side wall.

In any of these embodiments, an advantageous optional feature is that the first opening projects inside the chamber from a wall of the bellows more than the second opening.

In this way, as the bellows contracts, the first opening is closed off by one wall of the bellows before the same wall, or another wall of the bellows, reaches the second opening. This is a simple way of achieving the closure of the first opening while the second remains open.

According to an embodiment, the device comprises at least one of a bottom cover in which the first opening is defined, and a top cover in which the second opening is defined, said at least one cover being attached to the bellows.

This allows manufacturing these three parts of the device with different materials, each best suited to its specific function.

According to another embodiment of the invention, the vacuum regulator valve further comprises a seat associated to the first opening and a substantially rigid member coupled to the bellows such as to be displaced when the latter moves between the expanded condition and the contracted condition and projecting within the chamber, whereby when the bellows is in contracted condition the substantially rigid member rests on said seat thus closing the first opening and when the bellows is in expanded condition said rigid member is raised away from said seat thus leaving the first opening open.

Preferably, the substantially rigid member comprises an interior bore in fluid communication with the chamber, even when said rigid member is resting on said seat, and connectable to a suction conduit; the rigid member may have at least one side opening communicating the interior bore with the chamber.

According to an embodiment, the bellows comprises at least two pleats. This structure of the bellows has good elasticity, and thus allows fast and secure operation while maintaining a reduced dimension of the device.

In embodiments of the invention, the valve the bellows is arranged in a protective housing, said housing surrounding at least part of the bellows.

According to a further embodiment, the valve comprises a mechanical switch operable between a passive position, in which it allows the movement of the bellows between the expanded and the contracted conditions, and an active position, in which it prevents the bellows from adopting the contracted condition.

This allows locking the bellows in expanded condition, for example if it is desired to perform suction at high vacuum through the same device.

The device may comprise means for coupling auxiliary tubes to at least one of first opening and second opening such that the openings in the chamber are defined by the ends of such auxiliary tubes. Thus, the device may be attached very simply to existing suction bottles by attaching the tubes of the bottle to the device.

In such an embodiment, the valve may comprise means for coupling an auxiliary tube to the first opening, whereby, in use, the auxiliary tube projects inside chamber; preferably the valve comprises means for adjusting the projection of the auxiliary tube inside the chamber.

According to another aspect, the invention provides a pre-evacuated high vacuum suction bottle for removing body fluids, comprising a vacuum regulator valve as defined above.

In one embodiment, the bottle comprises a vacuum conduit connected to the first opening of the chamber of the vacuum regulator valve and a wound drainage conduit connected to the second opening of said chamber; alternatively, the bellows is attached to the bottle, with a neck projecting from the bottle inside the chamber and constituting said first opening of the chamber.

The bottle may further comprise means to selectively bypass the vacuum regulator device and connect the inside of the bottle to a wound drainage conduit. This allows the same bottle to be employed for high vacuum suction directly from the bottle or for low vacuum suction through the vacuum regulator valve, as desired.

### BRIEF DESCRIPTION OF THE FIGURES

Particular embodiments of the present invention will be described in the following, only by way of non-limiting example, with reference to the appended drawings, in which:
figure 1 is a schematic section view of a device according to an embodiment of the invention, with the bellows in expanded condition;
figure 2 is a view similar to that of figure 1, with the bellows in contracted condition;
figure 3 is a section view of a device according to a second embodiment of the intention;
figures 4 and 5 are side views, partially sectioned and taken from different angles, of a third embodiment of the device, attached to a suction bottle;
figure 6 is a partially sectioned section view of a further embodiment of the device, attached to a bottle; and
figure 7 shows an enlarged detail of figure 6.

### DESCRIPTION OF PREFERRED EMBODIMENTS

A vacuum regulator valve is described in the following, applied to the drainage of a wound by means of a pre-evacuated high vacuum suction bottle.

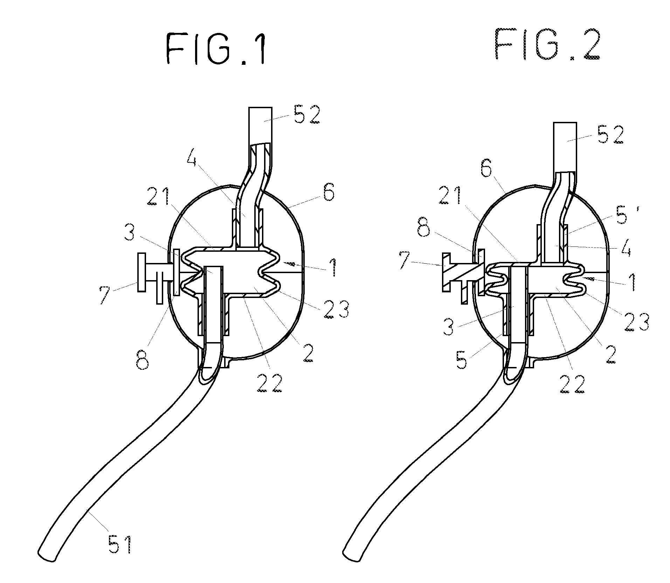

A first embodiment of the valve is shown in figures 1 and 2; as will be seen, this particular embodiment is appropriate to be arranged along the conduit between the high vacuum suction bottle and a wound to be drained.

The vacuum regulator valve shown in figures 1 and 2 has a bellows 1 which defines an inner chamber 2, said chamber having a first opening 3 and a second opening 4 arranged in opposite portions of the bellows.

In the figures, the bellows 1 comprises two flat end walls 21 and 22, namely a flat top wall 21 and a flat bottom wall 22, and a pleated side wall 23: the openings 3 and 4 are arranged on opposite end walls 21 and 22, but they could both be arranged on the top end wall 21 or on the bottom end wall 22.

Moulded in one piece with the bellows are a bushing 5 for coupling a first tube 51 to the first opening 3 and another bushing 5' for coupling a second tube 52 to the second opening 4. The first tube 51 is attached to the bushing 5 in such a way as to project inside the chamber, while the second tube 52 is attached to the bushing 5' flush or almost flush with the wall of the bellows.

In practice, the openings 3 and 4 in the chamber 2 are defined by the end of tubes 51 and 52, but of course this could be structured differently.

When in use, the first tube 51 is connected to a pre-evacuated high vacuum suction bottle for removing body fluids (not shown in figure 1), or other source of high vacuum. On the other hand, the second tube 52, when in use, is connected to the wound to be drained or other source of body fluid, thus constituting a suction conduit.

Furthermore, the valve also comprises a protective housing 6 surrounding the bellows, and a mechanical switch device 7 aligned with the pleated side wall of the bellows. The operation of the switch will be described later on.

The operation of the valve when employed to drain a wound with a high vacuum bottle is described in the following, starting from an expanded condition of the bellows 1 such as shown in figure 1.

Because of the high vacuum in the suction bottle and the communication between the bottle and the chamber 2 through the first opening 3, and because of the bellows being made from a deformable material such as rubber, the high vacuum causes the bellows pleats 7 to contract, reducing the volume of the chamber 2.

Hence, the top wall 21 of the bellows 1 arranged opposite the first opening 3 approaches said first opening until it contacts the mouth of the first tube 51, closing the opening 3, as shown in figure 2, where the bellows 1 is in its contracted condition. The second opening 4 remains open and in fluid communication with the chamber 2, because once the first opening 3 is closed, the level of vacuum in the chamber 2 cannot increase and the bellows does not contract further.

After the first opening 3 is closed, a vacuum level remains in the chamber 2, and causes a suction force through suction conduit 52, whereby fluids from the wound are suctioned towards the chamber 2 through said conduit.

However, the vacuum level in chamber 2 is subsequently reduced by said suction of fluids, such that the bellows 2 tend to expand again. When the bellows have expanded enough its top wall 21 loses contact with the first opening 3, which is therefore opened again. The fluids present in the chamber 2 are then suctioned towards the bottle by the higher vacuum in the bottle; and again the high vacuum in the bottle causes contraction of the bellows 1, whereby again the top wall 21 of the bellows closes the first opening 3.

These steps are repeated until the vacuum in the bottle in near atmospheric pressure, or the bottle fills up completely with fluid.

It will be understood that the pressure loss in the bellows 1 and the successive opening and closing of the first opening 3 cause the level of vacuum inside the chamber 2 to be lower than in the bottle, and thus the suction force on the wound to be relatively small compared to the force that would be exerted directly by the high vacuum in the bottle.

It is foreseen that the position of the first tube 51, i.e. the extent to which it projects inside the chamber 2, can be varied in order to provide a higher or lower level of vacuum inside the chamber 2.

It is also possible to provide some kind of reinforcement on the top wall 21, such as thickened portion (not shown) moulded together with the whole of the bellows 1, in order to improve the contact with the first opening 3 and guarantee a good closure.

The switch 7 arranged on one side of the protective housing 6 comprises a rigid plate 8 inside the housing, in correspondence with the pleated wall of the bellows. The switch can be rotated by hand and displaced between two positions: a passive position (shown in figure 1) in which the plate 8 does not interfere with the movement of the bellows, and an active position in which the plate 8 is displaced towards the right in figure 1, such as to press the pleated side wall 23 of the bellows 1 towards the right, against the inner wall of the housing 6.

In the active position of the switch 7 the side wall 23 of the bellows 1 is stiffened due to the pressure exerted on it by the plate 8 and the inner wall of the housing 6, such that the contraction of the bellows is hindered; as a consequence, the bellows are maintained in the expanded condition even under the effect of the high vacuum in the bottle.

Therefore, when the switch 7 is in the active position the bellows 1 does not operate and the high vacuum level from the bottle is in constant fluid communication with the suction conduit 52. This is useful because it allows the same bottle to be used for draining a wound at high pressure or at low pressure, as desired, simply by manually setting one position or other of the switch.

Although they have not been depicted in figures 1 and 2, the tubes 51 and/or 52 may be provided with appropriate cut-off clamps.

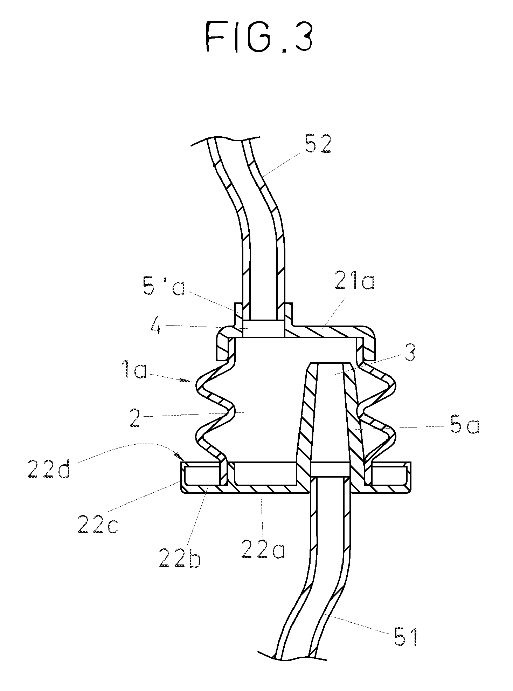

Figure 3 shows a second embodiment of the device. The operation is the same as described in relation to figures 1 and 2, but the physical structure of the device is somewhat different.

For instance, instead of a single part made of an elastomeric material forming the bellows and openings, in the embodiment of figure 3 the device comprises a substantially tubular bellows 1 a and two end covers 21 a, 22a firmly attached to the bellows.

The top end cover 21 a includes a bushing 5'a for inserting the second tube 52, while the bottom cover 22a includes a bushing 5a that projects inside the chamber 2 and forms the first opening 3 thereof. The first tube 51 is inserted in the first part of the bushing 5a of the bottom cover.

However, it would also be possible to form the bottom cover with a shorter bushing, or with an outward projecting bushing like that of the top end cover, and arrange the first tube 51 projecting inside the chamber to form the first opening, like in the embodiment of figures 1 and 2.

Manufacturing the device in different parts as in figure 3 allows to use the most appropriate material for each part, depending on its function: for example, the bellows can be manufactured in a polyurethane, which has very good elastic properties, and the top cover can be manufactured in natural or synthetic rubber or silicone, which is suitable for ensuring a tight closure of the first opening, or also of polyurethane. The top and bottom covers can be attached to the bellows by gluing.

The bottom cover 22a of the device of figure 3 is further provided with an annular flange 22b which projects beyond the bellows 1 a and ends in a vertical annular wall 22c. This arrangement forms a seat for attaching a suitable protective housing (not shown in figure 3), similar to that indicated with reference 6 in figures 1 and 2.

The vertical wall 22c of the bottom cover ends with an inwardly projecting rim or tooth 22d; this structure allows a suitably shaped housing to be snap-fitted to the device.

The devices of figures 1, 2 and 3 are intended to be inserted in the tube or conduit between the high vacuum bottle and the wound to be drained; however, other embodiments are possible. For example, the device can be attached to the bottle itself.

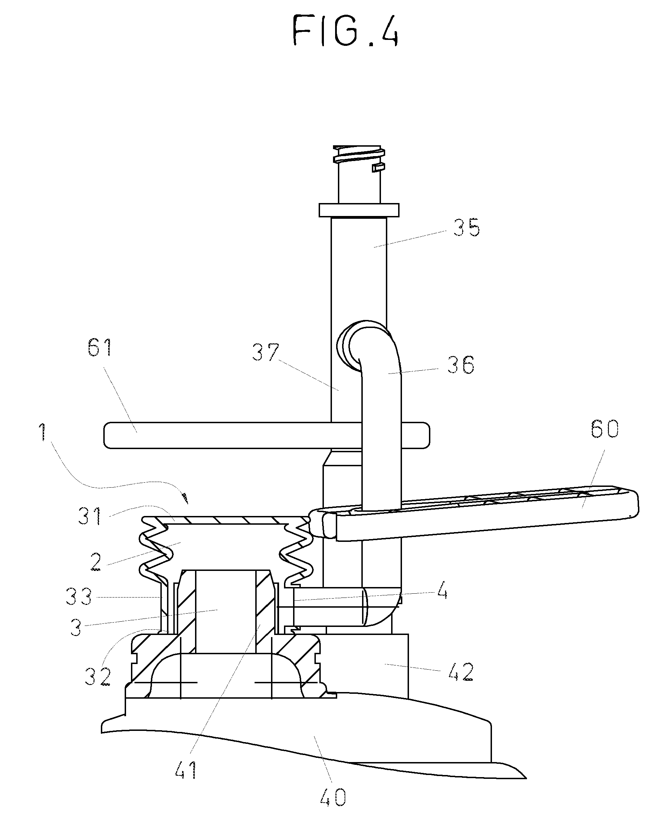

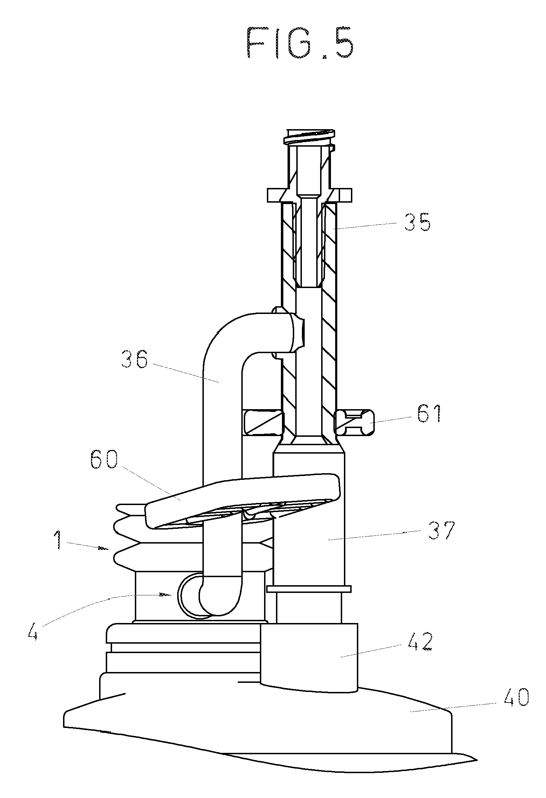

An embodiment in which the device is attached to the bottle is shown in figures 4 and 5. In these figures, the bellows 1 forming the chamber 2 is attached at its bottom flat wall 32 to a neck 41 of the high vacuum suction bottle 40. The bellows has a side wall 33 that is only partially pleated, with the unpleated portion of said side wall 33 surrounding the neck 41.

The first opening 3 of the chamber 2 is formed by the neck 41 of the bottle, which projects inside the bellows, such that the chamber 2 is in fluid communication with the bottle, while the second opening 4 is arranged in the unpleated portion of said side wall 33, at a distance from the neck 41 of the bottle, and is connected to a suction conduit that creates a fluid passage between the inside of the suction bottle 40 and the fluid source (i.e. a body wound), in a way that will be explained in more detail below.

Also in this case, when the device is in use the vacuum level inside the bottle 40, which is in communication with the chamber 2, leads the bellows 1 to switch to a contracted condition and the top flat end wall 31 of the bellows to approach the first opening 3 and close it, leaving an annular portion of the inner chamber 1 of the bellows in fluid communication with the second opening 4. The operation of the device is as described for the first embodiment.

By this functioning, a reduced vacuum level and a reduced suction force are achieved in the suction conduit connected with the second opening 4.

In this embodiment, the device is arranged and connected to the bottle in such a way to allow switching from suction with lowered vacuum level through the bellows, as described, and suction using high vacuum directly from the bottle 40, as will be described in the following.

As described above, the bottle 40 has a first neck 41 in communication through the chamber 2 with the second opening 4; as can be seen in figures 4 and 5, the second opening 4 is connected to a low vacuum tube 36.

Furthermore, the bottle has a second neck 42, directly connected to a high vacuum tube 37.

Tubes 36 and 37 and are both connected to a suction conduit 35, intended to be put in communication with a wound to be drained, or other fluid source. Furthermore, tubes 36 and 37 may each be closed off by means of respective cut-off clamps 60 and 61.

It will thus be understood that when one of the clamps 60, 61 is in closed condition and the other is in open condition, a fluid passage is created between the suction conduit 35 and one of the two necks 41, 42 of the bottle, allowing to switch between the high vacuum level of the bottle, or the low vacuum level created by the action of the valve device.

In other words, by selectively opening or closing clamps 60 and 61 the suction conduit 35 can be switched as desired between high vacuum suction (directly from the bottle 40 through neck 42 and tube 37) and low vacuum suction (through neck 41, bellows 1 and tube 36); the vacuum regulator device can thus be bypassed if desired to connect the inside of the bottle to the wound drainage conduit.

Of course the connection between tubes 36 and 37 and the suction conduit 35, as well as the means to selectively open or close each conduit, can be of any known kind.

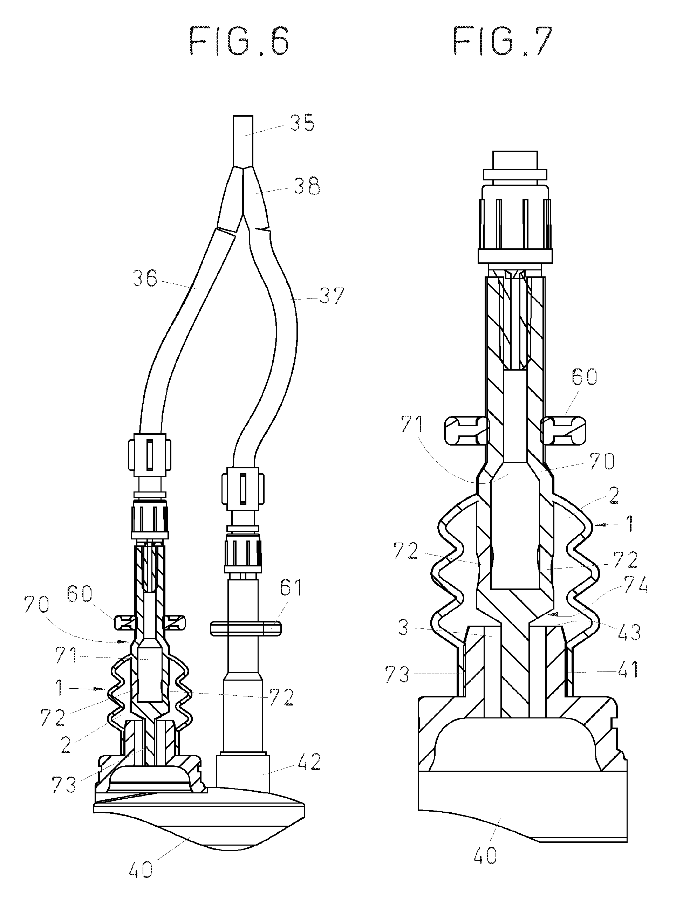

A further embodiment is shown in figures 6 and 7. In this case the bellows 1 is also attached to the neck 41 of the bottle 40 such that the first opening 3 is defined by said neck 41; however, the side wall of the bellows has no opening.

This embodiment foresees an elongate member 70, substantially rigid, associated to the bellows 1: the rigid member 70 is attached to the top wall of the bellows and projects inside the chamber 2. It will be understood that the rigid member 70 is displaced upwards when the bellows 1 expands and is displaced downwards when the bellows contracts. Figures 6 and 7 show the device with the bellows in expanded condition.

The rigid member 70 has in its upper part an interior bore 71, in communication with the low vacuum tube 36 as shown in the figure, and two side openings 72 through which the interior bore 71 is in communication with the chamber 2.

Furthermore, the lower end of the rigid member 70 has a tapered shoulder 74 ending in a portion 73 of smaller diameter that can extend into the neck 41 of the bottle.

The tapered shoulder 74 is appropriate to rest against a seat 43 formed on the mouth of the neck 41, such as to close the first opening 3 of the chamber 2, when the bellows 1 are in contracted condition.

With this arrangement, it will be understood that when the bellows 1 is in expanded condition the shoulder 74 of the rigid member 70 is raised from the seat 43 (best seen in figure 7), leaving the first opening 3 open and the chamber 2 in fluid communication with the high vacuum in the bottle 40; on the contrary, when the bellows 1 is in contracted condition the first opening 3 is closed by the shoulder 74 of the rigid member, and the chamber 2 is in fluid communication with the suction conduit 35 through the side opening 72, the bore 71 and the tube 36.

The operation of the device with the arrangement of figures 6 and 7 is much the same as described above for other embodiments.

Like in the embodiment of figures 4 and 5, in figure 6 the bottle has a second neck 42 connected to a high vacuum tube 37, with tubes 36 and 37 both connected to suction conduit 35 and provided with respective cut-off clamps 60 and 61, so also in this embodiment the suction force applied to drain the wound can be selected as desired.

In this case the tubes 36 and 37 are connected to the suction conduit 35 through a Y shaped connector 38.

Although only specific embodiments of the invention have been described above, the skilled man may substitute any particular element or feature by others that are technically equivalent, without departing from the scope of the present invention as defined in the appended claims.

For example, in some embodiments the device may further be provided with means to limit the possibility of deformation of the bellows on the side remote from the first opening, after this is closed; this could in some cases increase the life of the device and the reliability of its operation. Such means can for example be embodied by a substantially rigid member limiting the contraction of the bellows on the side remote from the first opening.

## Claims

1. A vacuum regulator valve, comprising a variable volume chamber (2) having a first opening (3) connectable to a vacuum source (40) and a second opening (4) connectable to a suction conduit (52; 36), such that, in use, the chamber (2) may be put in fluid communication with the vacuum source (40) and vacuum may be generated in the chamber to cause suction through the suction conduit (52; 36), **characterised in that** the valve comprises a bellows (1; 1 a) with at least one pleat, arranged to define the chamber (2) and operable to move, in use, between an expanded condition in which the first opening (3) of the chamber (2) remains open, and a contracted condition in which it causes the closure of the first opening (3).

2. A valve according to claim 1 wherein in the contracted condition of the bellows (1) a portion of its inner wall rests against the first opening (3), closing it.

3. A valve according to claim 2 wherein the portion of the inner wall of the bellows (1) which closes the first opening (3) comprises a thickened section.

4. A valve according to any of claims 1 to 3 wherein the bellows (1) comprises two substantially flat end walls (21,22; 31,32) and a side wall (23; 33) which is at least partly pleated.

5. A valve according to claim 4 wherein the first opening (3) and second opening (4) are defined on the same flat end wall (21,22).

6. A valve according to claim 4 wherein the first opening (3) and second opening (4) are defined on opposite flat end walls (21,22).

7. A valve according to claim 4 wherein the first opening (3) is defined on one flat end wall (32) and the second opening (4) is defined on the side wall (33).

8. A valve according to any of claims 4 to 7 wherein the first opening (3) projects inside the chamber (2) from the end wall more than the second opening (4).

9. A valve according to claim 1, comprising at least one of a bottom cover (22a) in which the first opening (3) is defined and a top cover (21 a) in which the second opening (4) is defined, said at least one cover (21 a,22a) being attached to the bellows (1 a).

10. A valve according to claim 1 further comprising a seat (43) associated to the first opening (3) and a substantially rigid member (70) coupled to the bellows (1) such as to be displaced when the latter moves between the expanded condition and the contracted condition and projecting within the chamber (1), whereby when the bellows (1) is in contracted condition the rigid member (70) rests on said seat (43) thus closing the first opening (3) and when the bellows (1) is in expanded condition the rigid member (70) is raised away from said seat (43) thus leaving the first opening (3) open.

11. A valve according to claim 10 wherein the substantially rigid member (70) comprises an interior bore (71) in fluid communication with the chamber (2), even when said rigid member (70) is resting on said seat (43), and connectable to a suction conduit (36).

12. A valve according to claim 11 wherein the substantially rigid member (70) has at least one side opening (72) communicating the interior bore (71) with the chamber (2).

13. A valve according to any of the preceding claims wherein the bellows (1) comprises at least two pleats.

14. A valve according to any of the preceding claims wherein the bellows (1) is arranged in a protective housing (6), said housing surrounding at least part of the bellows (1).

15. A valve according to any of the preceding claims comprising a mechanical switch (7) operable between a passive position, in which it allows the movement of the bellows (1) between the expanded and the contracted conditions, and an active position, in which it prevents the bellows (1) from adopting the contracted condition.

16. A valve according to any of the preceding claims comprising means (5,5') for coupling auxiliary tubes (51,52) to at least one of first opening (3) and second opening (4) such that the openings (3,4) in the chamber (2) are defined by the ends of such auxiliary tubes (51,52).

17. A valve according to claim 16 comprising means for coupling an auxiliary tube (51) to the first opening (3), whereby, in use, the auxiliary tube (51) projects inside chamber (2).

18. A valve according to claim 17 comprising means for adjusting the projection of the auxiliary tube (51) inside the chamber (2).

19. A pre-evacuated high vacuum suction bottle for removing body fluids, **characterised in that** it comprises a valve as claimed in any of the preceding claims.

20. A bottle according to claim 19 further comprising a vacuum conduit (51) connected to the first opening (3) of the chamber (2) of the valve and a wound drainage conduit (52) connected to the second opening (4) of said chamber (2).

21. A bottle according to claim 19 wherein the bellows (1) is attached to the bottle, with a neck (41) projecting from the bottle (40) inside the chamber (2) and constituting said first opening (3) of the chamber (2).

22. A bottle according to claim 21, further comprising means to selectively bypass the vacuum regulator device and connect the inside of the bottle (40) to a wound drainage conduit (35).
